(19) Europäisches Patentamt

European Patent Office

Office européen des brevets

(11) Publication number: **0 186 946**
**B1**

(12) **EUROPEAN PATENT SPECIFICATION**

(45) Date of publication of patent specification: **03.01.90**

(21) Application number: **85307592.7**

(22) Date of filing: **21.10.85**

(51) Int. Cl.⁵: **G 01 N 33/546,**
**G 01 N 33/569**

(54) **Passive agglutination assay for pseudorabies antibody.**

(30) Priority: **22.10.84 US 663853**

(43) Date of publication of application:
**09.07.86 Bulletin 86/28**

(45) Publication of the grant of the patent:
**03.01.90 Bulletin 90/01**

(84) Designated Contracting States:
**AT BE CH DE FR GB IT LI LU NL SE**

(73) Proprietor: **VIRAL ANTIGENS, INC.**
**5171 Wilfong Road**
**Memphis Tennessee 38134-5611 (US)**

(72) Inventor: **Dorsett, Preston H.**
**3865 Brunswick Road**
**Memphis Tennessee 38134 (US)**

(74) Representative: **Bizley, Richard Edward et al**
**BOULT, WADE & TENNANT 27 Furnival Street**
**London EC4A 1PQ (GB)**

(56) References cited:
**EP-A-0 054 249**
**EP-A-0 133 200**
**US-A-4 264 766**

**CHEMICAL ABSTRACTS, vol. 90, no. 1, 1st January 1979, page 411, column 1, abstract no. 4234k, Columbus, Ohio, US; F. MOUTOU et al.: "Application of an enzyme linked immunosorbent assay for diagnosis of Aujeszky's disease in swine", & VET. REC. 1978, 103(12), 264**

(56) References cited:

**CHEMICAL ABSTRACTS, vol. 97, no. 3, 19th July 1982, page 538, column 2, abstract no. 21812j, Columbus, Ohio, US; C.C.C. YEN et al.: "Application of the enzyme-linked immunosorbent assay (ELISA) to the detection of antibody of pseudorabies in swine", & CHUNG-HUA MIN KUO SHOU I HSUEH HUI TSA CHIH, 1981, 7(2), 103-108**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates generally to materials and methods useful for the detection of antibodies; in particular, the invention relates to immunoreagents and immunodiagnostic tests for the detection of pseudorabies antibody in serum samples.

Pseudorabies is an infectious bulbar paralysis also known as mad itch or Aujeszky's disease. Its causitive agent is pseudorabies virus which is a herpes virus *suis* belonging to the family of Herpesviridae. Pseudorabies is mainly a disease of swine and cattle.

Swine, which is probably the natural host reservoir, infected with pseudorabies often show no symptoms. Infrequently, however, adult swine develop fever and neurological symptoms. Although many infected swine show no symptoms, the virus infection becomes latent and remains for the life of the animal.

In cattle, pseudorabies is a rapidly fatal non suppurative encephalomyelitis characterized by intense pruritus and self mutilation. Affected cattle generally die within three days of the onset of clinical signs.

Since swine are generally regarded as the natural host and usual reservoir of pseudorabies virus, it is imperative to diagnose psueudorabies in swine before the swine intermingle with cattle. Bovine pseudorabies is prevented only by keeping swine separate from cattle. Although vaccines have been developed which are effective for swine, there is no effective vaccine for cattle.

A serological test for the detection of antibodies to pseudorabies virus is a valuable aid in identifying persistently infected pigs. However, current methods for antibody detection of pseudorabies are time consuming and often not sensitive enough to detect very low levels of antibody. In testing swine, the microtiter neutralization (SVN) test is the commonly employed method (Hill et al, *Proceedings of the American Association of Veterinary Laboratory Diagnosis*, 20:375—390 (1977)). Significant disadvantage is incurred with the SVN test. The SVN test involves a three to four hour set-up time plus a forty-eight hour incubation time. Another serious drawback of the SVN test is that it involves a biohazard risk associated with the use of infectious virus.

Accordingly, there remains a need for an improved immunodiagnostic test for pseudorabies virus antibody.

The present invention provides a latex composition and methods for its use to detect the presence of pseudorabies virus antibody in a biological sample.

The latex composition of this invention is composed of pseudorabies virus antigens physically adsorbed onto or bound to discrete latex particles. The pseudorabies virus antigens are the products of disrupted and solubilized pseudorabies virus. The disrupted pseudorabies virus products provide a variety of pseudorabies virus antigens which are selectively immunoreactive with pseudorabies virus antibody. In particular, the use of the disrupted virus avoids the biohazard associated with infectious virus. Moreover, adsorption of the disrupted, solubilized virus antigens to the latex particles results in a smooth latex dispersion in contrast to the aggregated, clumped latex particles which result after adsorption of whole intact virus.

In the methods of this invention, the latex composition is employed in a direct agglutination assay to detect the presence of pseudorabies virus antibody in a biological sample. The test involves admixing a biological sample, such as serum, with the latex composition. The admixture is incubated under conditions favorable for immunoreaction. Thereafter the admixture is examined macroscopically for agglutination or clumping of the latex particles. Agglutination is indicative of the presence of pseudorabies virus antibody in the biological sample.

The following discussion is in terms of the preferred embodiments of this invention, which represent the best mode known to the Applicants at the time of this application.

In accordance with this invention a latex composition is provided which is of particular value in a direct agglutination assay for pseudorabies virus antibody. The latex composition of this invention consists essentially of disrupted and solubilized pseudorabies virus products supported on a latex carrier particle.

### Latex

As used within the context of this invention, latex carrier particles include latex polymers which are water insoluble, have a part-cle size (diameter) in the range of about 0.05 micron to about 2 microns and more preferably of about 0.9 micron, have a specific gravity near that of water so that they can remain in aqueous suspension, and are inert with respect to immunological reactions. Further, the latex particles must have sufficient repulsive forces after adsorption of the pseudorabies virus antigen so as to prevent their aggregation in the absence of an immunological reaction.

Typical suitable latex carrier particles are those supplied commercially as an aqueous latex suspension, usually in concentrations of about 20 to about 60% solids. Many types of latex are suitable for use in this invention so long as they meet the criteria listed above.

Typical suitable latex carrier polymers are carboxylated polystyrenes, acrylic acid polymers, methacrylic acid polymers, acrylonitrile butadiene styrenes, polyvinyl acetate acrylates, polyvinyl pyridines and vinyl chlorideacrylates, for example. Some commercially available latexes suitable for use in this invention are AMSCO Res 4150, AMSCO Res. 3011 (American Mineral Spirits Co); Dow Latex 815; 816; 620; or 859 (The Dow Chemical Co.); Hycar 1512, Hycar 1877x8, Hycar 2600x 120 (Goodrich Chemical Co.); Gelva 900, Lytron 612, Lytron 624 (Monsanto Co.); Rhoplox LC 403216, Amberlite Ultrafine (Rohn and Hass Co); and Bacto-latex 0.81 (Difco Laboratories). The above trade marks are registered by the respective companies listed.

Pseudorabies Virus Antigen

Further in accordance with this invention partially purified pseudorabies virus antigens are provided for attachment to the latex particles. The pseudorabies virus antigens are the product or disrupted and solubilized pseudorabies virus. A preferred method for preparing the virus antigens is described below.

The pseudorabies whole virus is first produced in tissue culture, for which a procedure known in the art may be used. In particular; pseudorabies virus can be grown in swine testes cells, primary pig kidney cells, a PK—15 cell line, primary rabbit kidney cells, and a swine fibroblast cell line, MDBK.

After filtration and concentration of the pseudorabies virus-containing fluid obtained from cell culture, the pseudorabies virus is then purified. More particularly, after filtration and concentration, the virus is contacted with hydroxyl apatite slurry in an aqueous suspension having an ionic strength which is great enough to minimize or prevent adsorption of the virus by the gel, and which is low enough to allow some of the non-virus proteins to be adsorbed by the hydroxyl apatite gel. The ionic strength is maintained by the use of a phosphate buffer, with the phosphate ions being present at a molarity of about 0.001M. This ionic strength provides effective adsorption of non-virus proteins and nucleic acids, without significant adsorption of the virus. The phosphate molarity in most cases is between .001 to .005M.

In addition, the adsorption is conducted at a pH in the order of from 6 to 10, most generally in the order of from 8 to 9. The pH of the solution is maintained by the use of a suitable buffer. The adsorption may be conducted in the presence of EDTA at a concentration from .01M to .0001M. EDTA as well as other chelating agents increases adsorption of non-viral proteins and nucleic acids, and aids in minimizing the adsorption of viral proteins.

In proceeding with the purification, the high molecular weight proteins and nucleic acids are adsorbed by the gel to thereby separate the virus protein from the non-viral proteins having similar molecular weights.

After such adsorption, the lower molecular weight proteins still remaining in the fluid may be separated, e.g. by conventional procedures. Thus, for example, further separation may be accomplished by centrifugation through a barrier layer or cushion as known in the art. In particular, the virus protein is centrifuged through a suitable barrier layer such as sucrose, glycerol, cesium chloride or cesium sulfate, for example, with the lower molecular weight proteins remaining above the barrier, and the virus being centrifuged through the barrier, as a separate layer. The fluid containing the low molecular weight proteins and the barrier layer is then removed leaving a virus protein essentially free of non-virus proteins, nucleic acids, lipids, and the like. In general, the purified virus contains less than 1%, most generally less than 0.1% of non-virus lipids, nucleic acids and proteins.

Such purified virus may then be treated with a surfactant to disrupt the virus and effect solubilization to provide soluble viral antigens.

Pseudorabies virus antigen is prepared from intact pseudorabies virus by treating purified whole pseudorabies virus with a surfactant or detergent which disrupts the virus to provide the soluble pseudorabies virus antigen, without destroying its antigenic characteristics. The detergent is employed in an amount that is sufficient to disrupt and solubilize the whole virus without destroying antigenicity. In general the surfactant to virus weight ratio is from 0.2:1 to about 5:1, preferably from about 0.4:1 to 1:1.

The surfactant or detergent which is used for disrupting the whole pseudorabies virus may be any one of a wide variety of surfactants or detergents, including cationic, anionic and nonionic surfactants. Such surfactants are well known in the art, and as representative examples, there may be mentioned alkali metal salts of sulfates, soaps, sulfated or sulfonated oils, various amines, quaternary salts, condensation products with ethylene oxide, etc. Preferred detergents for such use are alkali (lithium or sodium) dodecyl sulfate, sulfobetain, deoxylcholate and laurolylsarcosine (Sarcosyl).

The treatment of the purified virus is effected at a temperature which does not denature the virus proteins, with such temperature generally not exeeding about 37°C, with a temperature from 20° to 37°C being most convenient. Similarly, the pH is selected so as to maintain stability, with the pH being generally at 8.5, with the optimum pH generally being in the order of from 8.0 to about 9.0.

The treatment of the purified virus with the surfactant is for a period of time sufficient to disrupt the virus and effect solubilization thereof. In general, such disruption and solubilization can be accomplished in time periods in the order of from 5 to 120 minutes, however, in some cases longer or shorter times may be applicable.

Latex supported Pseudorabies Virus Antigen Composition

After recovering the disrupted pseudorabies virus products they are attached by passive adsorption to the latex carrier particles. The antigen is adsorbed to the latex in an effective amount to facilitate the agglutination assay, but in an amount low enough to prevent bridging of the antibody on a single particle. In general, the weight ratio of soluble antigen to support is from 1:30 to 1:200; and 1:50 to 1:100 is the preferred range.

In addition to passive adsorption techniques, the disrupted pseudorabies virus products can be covalently bound to the latex carrier. In particular, U.S. Patents 4,264,766, and 4,210,723 provide numerous latex particles having active groups which are capable of forming covalent linkages with immunologically active substances such as the disrupted pseudorabies virus antigen products of this invention.

For example, such covalent linkages include carboxyl groups, amine groups or groups convertible into them, e.g. —COOH—$CONH_2$, a nitrile group, a primary amine or a secondary amine group.

In accordance with yet another embodiment of this invention, after the antigen is adsorbed onto the latex particles, the support, including the adsorbed antigen, is further coated with an immunologically inert protein, for example bovine serum albumin or ovalbumin, which does not react with the pseudorabies antigen or adversely affect the subsequent immunochemical reaction with the antibody.

The pseudorabies virus antigen sensitized particles prepared in accordance with this invention are suitable for use in a kit and assay for pseudorabies virus antibody by a direct agglutination procedure. Such a kit may include, in addition to the sensitized pseudorabies virus particles in a suitable container, a reactive serum control containing pseudorabies antibody, and a non-reactive serum control in suitable containers therefor.

In accordance with a preferred embodiment, in addition to the reagents, there is provided a test slide on which the assay is effected. The test slide has a flat testing surface which includes suitably marked areas (for example, a test circle) for placing one or more samples to be assayed, as well as suitably marked areas for each of the serum controls. The test slide and reagents may be included in a single kit package.

Direct Agglutination Assay

The antigen-antibody reaction is the basis for all immunological assays. Antibodies are produced by mammals in response to the presence of a foreign substance called an antigen, usually a protein. This normal body response to a foreign protein, including bacterial and viral agents, has led to the development of a number of techniques useful to diagnose mammalian infection or disease.

In vitro tests for detecting antibody in a biological sample are generally carried out by contacting the antigen with a biological sample. If the suspected antibody is present, the resulting antigen-antibody reaction can be demonstrated by precipitation or agglutination of the antigen-antibody complex. This antigen-antibody reaction is usually very difficult to detect visually. By binding the test antigens to a particulate carrier, such as latex, the subsequent antigen-antibody agglutination reaction becomes readily visible. Agglutination is characterized by the macroscopic clumping of the latex particles from an otherwise smooth suspension.

In a direct latex agglutination test for the detection of pseudorabies antibody in a biological sample, such as swine serum, the biological sample is mixed with a suspension of the latex supported pseudorabies antigen. If pseudorabies antibody is present in the biological sample, it will react with the antigen to form a precipitate or aggregate of the latex particles. If no pseudorabies antibody is present, the latex suspension will keep its appearance as a smooth suspension.

To facilitate specific antibody-antigen reaction, swine serum can, prior to the assay, be adsorbed with kaolin, bentonite, apatite, talc, or charcoal to reduce its lipid content and nonspecific binding agents. Alternatively, the serum can be used at a 1:4 to 1:10 dilution which eliminates most nonspecific reactions.

Example I
Production and Purification of Pseudorabies Virus

Sullivan strain pseudorabies virus was originally isolated from a natural infection of a pig in Camden, Indiana. The virus was obtained from Dr. D. P. Gustafson, Purdue University. At Wisconsin State University, the virus had been passaged once in adult pig thyroid cell monolayers. Afterwards, it was plaque purified three times in a continuous culture swine testes cells. Plaque purified virus in the fourth passage in swine testes cells were used for these experiments.

Confluent roller cultures ($680cm^2$) of swine testes cells were innoculated with approximately 0.01 PFU of pseudorabies virus per cell and maintained in a standard culture medium (Medium 199) containing 0.025 M hepes buffer, pH 7.6, and 5% (vol/vol) of tryptose phosphate broth. The cells were incubated from 48—96 hours at 36 ± 1°C. Harvested by freezing at −70°C, the cells were next thawed to disrupt residual cells. Afterward the thawed material was centrifuged differentially, first at 4000 × g then at 16,000 × g each for 15—20 min. The supernatant was concentrated in an Amicon hollow fiber dialyzer-concentrator to 1/6 the original volume. After clarification at 5,000 × g, the concentrate was made to contain .001M EDTA, the pH was adjusted to 8.5 at 22°C room temperature, and the concentrate was clarified at 16,000 × g. Next 1/10 volume of hydroxylapatite suspension was added, and the slurry was incubated at 4°C with mixing, for one hour. The hydroxylapatite was removed by centriguation at 16,000 × g for 15 minutes, after which 30 ml of the concentrate was layered over 9 ml of 69% (wt/wt) glycerol in a Beckman SW28 tube. The virus was sedimented at 82,000 × g for 16 hours at 4°C, and the resultant pellet was resuspended in NTE buffer: 0.01M Tris containing 0.12M NaCl and 0.001M EDTA, pH 8.5. The purified virus was stored at −70°C.

Example II
Solubilization of Purified Virus

The purified virus was dissolved in an equal volume of NTE buffer and the protein content was determined by the O.D. 260/280nm ratio. The virus was disrupted and solubilized by treatment with sodium dodecyl sulfate (SDS) at a concentration of 0.5 mg SDS per 1 mg of viral protein (w/w) for 30 minutes at 36—37°C.

## Example III
### Preparation of Sensitized Latex Commercial suspensions of polystyrene latex (0.9 micron diameter particles) were washed four times with 50 volumes each of 0.01M carbonate pH 9.5 buffer and were resuspended in the carbonate buffer to provide 3% solids (vol/vol). The solubilized virus was added directly to the latex with mixing at a ratio of 0.4 mg of purified protein per ml of 3% latex. The suspension was mixed by tumbling for 16 hours at 4°C. The sensitized latex was diluted with an equal volume of phosphate buffered saline. The latex was collected by centrifugation at 8000 × g for 10 minutes and the pellet was resuspended to form a 0.6% suspension in 1% bovine serum albumin in phosphate buffered saline (BSA—PBS) containing 0.05% polyoxyethylene sorbitan monolaurate surface active agent (Tween — registered trade mark of ICI United States, Inc. — 20) and 0.02% gentamicin. The sensitized latex was sonicated in 5 ml aliquots for 1 second at full power on a Bronson Model S75 Sonifier.

## Example IV
### Latex Agglutination Test for Pseudorabies Virus Antibodies

Glass plates with 1.4 cm fused circles were employed. Serial 2-fold dilutions of serum were prepared in 1% BSAPBS containing Tween 20 and 50 µl of each dilution was placed in separate circles, and spread to cover the circle. After adding 15—20 µl (1 drop) of sensitized latex, the serum and latex suspension was mixed by hand or rotated at 100 rpm for 5—8 minutes. The slide was read macroscopically in the wet state. The presence of antibody against pseudorabies virus was evidenced by visible agglutination.

## Example V

A serum sample was mixed with an equal volume of 25% kaolin suspension. The mixture was allowed to stand at room temperature for 20 minutes. The kaolin was removed by centrifugation at 2000 rpm for 10 minutes. The resultant supernatant fluid was a 1:2 dilution of the serum. With a micropipettor, 25 µl of the supernatant fluid was placed into a fused circle on the glass slide. With the micropipettor, 25 µl of the sensitized latex reagent was then added to the same circle. The slide was rotated on a rotator at approximately 100 rpm for 8 minutes, while placed under a humidifier cover. Immediately following rotation, the slide was read macroscopically in the wet state under a high intensity incandescent lamp. Positive tests were noted as a clumping of the latex. In negative tests, the latex remained in smooth suspension.

## Claims for the Contracting States: BE CH DE FR GB IT LI LU NL SE

1. A latex composition comprising discrete latex particles having attached thereon disrupted pseudorabies virus products, said products providing antigens which are selectively immunoreactive with pseudorabies virus antibody.

2. A latex composition as claimed in claim 1 which is dispersed in an aqueous suspension.

3. A latex composition as claimed in claim 1 or claim 2 wherein the weight ratio of disrupted pseudorabies virus products to latex particle is from 1:30 to 1:200.

4. A latex composition as claimed in any one of the preceding claims which further includes bovine serum albumin, ovalbumin or another immunologically inert protein adsorbed to the latex particles.

5. A latex composition as claimed in any one of the preceding claims wherein the latex is polystyrene latex.

6. A latex composition as claimed in any one of the preceding claims wherein the latex particle ranges from 0.05 to 2 microns diameter.

7. A latex composition as claimed in any one of the preceding claims wherein the disrupted pseudorabies virus products are passively adsorbed to the latex particles, or are covalently bound to the latex particles.

8. A latex composition as claimed in any one of the preceding claims wherein the disrupted pseudorabies virus products are produced from surfactant disrupted and solubilized pseudorabies virus.

9. A method for detecting the presence of pseudorabies virus antibody in a biological sample, comprising:

mixing the biological sample with a latex composition comprising disrupted pseudorabies virus products attached to latex particles dispersed in a liquid suspension, said pseudorabies virus products providing antigens which are selectively immunoreactive with pseudorabies virus antibody, as claimed in Claim 1;

incubating the mixture of the latex composition and biological sample for a time sufficient to allow an immunoreaction; and

determining if agglutination occurs, whereby agglutination indicates the presence of pseudorabies virus antibody in the biological sample.

10. A method as claimed in claim 9 wherein the biological sample is swine or other serum or plasma.

11. A method as claimed in any one of the preceding claims wherein the biological sample is pretreated with kaolin, talc, charcoal or apatite to remove non-specific agglutinins and/or diluted with an immunologically inert diluent prior to mixing with the latex composition.

**Claims for the Contracting State: AT**

1. A method for making a latex composition comprising discrete latex particles having attached thereon disrupted pseudorabies virus products, said products providing antigens which are selectively immunoreactive with pseudorabies virus antibody, the method comprising contacting discrete latex particles with disrupted pseudorabies virus products.

2. A method as claimed in claim 1 wherein the latex particles are dispersed in an aqueous liquid.

3. A method as claimed in claim 1 or claim 2 wherein the weight ratio of disrupted pseudorabies virus products to latex particle is from 1:30 to 1:200.

4. A method as claimed in any one of the preceding claims which further comprises adsorbing bovine serum albumin, ovalbumin or another immunologically inert protein to the latex particles after they are contacted with the disrupted pseudorabies virus products.

5. A method as claimed in any one of the preceding claims wherein the latex is polystyrene latex.

6. A method as claimed in any one of the preceding claims wherein the latex particle ranges from 0.05 to 2 microns diameter.

7. A method as claimed in any one of the preceding claims wherein the disrupted pseudorabies virus products are passively adsorbed to the latex particles or are covalently bound to the latex particles, and/or the disrupted pseudorabies virus products are produced by disrupting and solubilizing pseudorabies virus with surfactant.

8. A method for detecting the presence of pseudorabies virus antibody in a biological sample, comprising

mixing the biological sample with a latex composition comprising disrupted pseudorabies virus products attached to latex particles dispersed in a liquid suspension, said pseudorabies virus products providing antigens which are selectively immunoreactive with pseudorabies virus antibody, as claimed in Claim 1;

incubating the mixture of the latex composition and biological sample for a time sufficient to allow an immunoreaction; and

determining if agglutination occurs, whereby agglutination indicates the presence of pseudorabies virus antibody in the biological sample.

9. A method as claimed in claim 8 wherein the biological sample is swine or other serum or plasma.

10. A method as claimed in claim 8 or claim 9 wherein the biological sample is pretreated with kaolin, talc, charcoal or apatite to remove non-specific agglutinins and/or diluted with an immunologically inert diluent prior to mixing with the latex composition.

**Patentansprüche für die Vertragsstaaten: BE CH DE FR GB IT LI LU NL SE**

1. Latex-Zusammensetzung, umfassend diskrete Latexpartikel, die gebrochene Pseudorabies-Virusprodukte daran gebunden enthalten, wobei die genannten Produkte Antigene liefern, die mit Pseudorabies-Virus-Antikörper selektiv immunoreaktiv sind.

2. Latex-Zusammensetzung gemäss Anspruch 1, dadurch gekennzeichnet, dass sie in einer wässrigen Suspension dispergiert ist.

3. Latex-Zusammensetzung gemäss Anspruch 1 oder 2, dadurch gekennzeichnet, dass das Gewichtsverhältnis von gebrochenen Pseudorabies-Virusprodukten zu Latexpartikeln von 1:30 bis 1:200 beträgt.

4. Latex-Zusammensetzung gemäss jedem der vorhergehenden Ansprüche, dadurch gekennzeichnet, dass sie ferner Rinderserumalbumin, Ovalbumin oder ein anderes immunologisch inertes Protein, adsorbiert an die Latexpartikel, enthält.

5. Latex-Zusammensetzung gemäss jedem der vorhergehenden Ansprüche, dadurch gekennzeichnet, dass der Latex ein Polystyrol-Latex ist.

6. Latex-Zusammensetzung gemäss jedem der vorhergehenden Ansprüche, dadurch gekennzeichnet, dass die Latexpartikel einen Durchmesser von 0,05 bis 2μm aufweisen.

7. Latex-Zusammensetzung gemäss jedem der vorhergehenden Ansprüche, dadurch gekennzeichnet, dass die gebrochenen Pseudorabies-Virusprodukte an die Latexpartikel passiv adsorbiert oder an die Latexpartikel kovalent gebunden sind.

8. Latex-Zusammensetzung gemäss jedem der vorhergehenden Ansprüche, dadurch gekennzeichnet, dass die gebrochenen Pseudorabies-Virusprodukte aus durch oberflächenaktives Mittel gebrochenen und solubilisierten Pseudorabies-Viren hergestellt werden.

9. Verfahren zum Nachweis von Pseudorabies-Virus-Antikörpern in einer biologischen Probe, wobei man:

die biologische Probe mit einer Latex-Zusammensetzung mischt, die gebrochene Pseudorabies-Virusprodukte, gebunden an in einer flüssigen Suspension dispergierte Latexpartikel, umfasst, wobei die genannten Pseudorabies-Virusprodukte Antigene liefern, die mit Pseudorabies-Virus-Antikörper selektiv immunoreaktiv sind, wie dies in Anspruch 1 beansprucht ist;

die Mischung der Latex-Zusammensetzung und der biologischen Probe über einen für eine Immunoreaktion hinreichenden Zeitraum inkubiert; und

durch Agglutination die Bestimmung durchführt, wobei Agglutination das Vorliegen von Pseudorabies-Virus-Antikörper in der biologischen Probe anzeigt.

10. Verfahren gemäss Anspruch 9, dadurch gekennzeichnet, dass die biologische Probe Schweine- oder ein anderes Serum oder Plasma ist.

11. Verfahren gemäss jedem der vorhergehenden Ansprüche, dadurch gekennzeichnet, dass die biologische Probe zur Entfernung nicht-spezifischer Agglutinine mit Kaolin, Talk, Kohle oder Apatit vorbehandelt und/oder vor dem Vermischen mit der Latex-Zusammensetzung mit einem immunologisch inerten Verdünnungsmittel verdünnt wird.

## Patentansprüche für den Vertragsstaat: AT

1. Verfahren zur Herstellung einer Latex-Zusammensetzung, die diskrete Latex-Partikel umfasst, die gebrochene Pseudorabies-Virusprodukte daran gebunden enthalten, welche Antigene liefern, die mit Pseudorabies-Virus-Antikörper selektiv immunoreaktiv sind, wobei man diskrete Latexpartikel mit gebrochenen Pseudorabies-Virusprodukten in Kontakt bringt.

2. Verfahren gemäss Anspruch 1, dadurch gekennzeichnet, dass die Latexpartikel in einer wässrigen Flüssigkeit dispergiert sind.

3. Verfahren gemäss Anspruch 1 oder 2, dadurch gekennzeichnet, dass das Gewichtsverhältnis von gebrochenen Pseudorabies-Virusprodukten zu Latexpartikeln von 1:30 bis 1:200 beträgt.

4. Verfahren gemäss jedem der vorhergehenden Ansprüche, dadurch gekennzeichnet, dass man ferner Rinderserumalbumin, Ovalbumin oder ein anderes immunologisch inertes Protein an die Latexpartikel adsorbiert, nachdem sie mit den gebrochenen Pseudorabies-Virusprodukten in Kontakt gebracht sind.

5. Verfahren gemäss jedem der vorhergehenden Ansprüche, dadurch gekennzeichnet, dass der Latex Polystyrol-Latex ist.

6. Verfahren gemäss jedem der vorhergehenden Ansprüche, dadurch gekennzeichnet, dass die Latexpartikel Durchmesser von 0,05 bis 2 μm aufweisen.

7. Verfahren gemäss jedem der vorhergehenden Ansprüche, dadurch gekennzeichnet, dass die gebrochenen Pseudorabies-Virusprodukte an die Latexpartikel passiv adsorbiert oder an die Latexpartikel kovalent gebunden werden, und/oder dass die gebrochenen Pseudorabies-Virusprodukte erzeugt werden, indem man das Pseudorabies-Virus mit oberflächenaktivem Mittel bricht und solubilisiert.

8. Verfahren zum Nachweis von Pseudorabies-Virus-Antikörpern in einer biologischen Probe, wobei man:

die biologische probe mit einer Latex-Zusammensetzung mischt, die gebrochene Pseudorabies-Virusprodukte, gebunden an in einer flüssigen Suspension dispergierte Latexpartikel, umfasst, wobei die genannten Pseudorabies-Virusprodukte Antigene liefern, die mit Pseudorabies-Virus-Antikörper selektiv immunoreaktiv sind, wie dies in Anspruch 1 beansprucht ist;

die Mischung der Latex-Zusammensetzung und der biologischen Probe über einen für eine Immunoreaktion hinreichenden Zeitraum inkubiert; und

durch Agglutination die Bestimmung durchführt, wobei Agglutination das Vorliegen von Pseudorabies-Virus-Antikörper in der biologischen Probe anzeigt.

9. Verfahren gemäss Anspruch 8, dadurch gekennzeichnet, dass die biologische Probe Schweine- oder ein anderes Serum oder Plasma ist.

10. Verfahren gemäss Anspruch 8 oder 9, dadurch gekennzeichnet, dass die biologische Probe zur Entfernung nicht-spezifischer Agglutinine mit Kaolin, Talk, Kohle oder Apatit vorbehandelt und/oder vor dem Vermischen mit der Latex-Zusammensetzung mit einem immunologisch inerten Verdünnungsmittel verdünnt wird.

## Revendications pour les Etats contractants: BE CH DE FR GB IT LI LU NL SE

1. Une composition de latex comprenant des particules discrètes de latex auxquelles sont liés des produits du virus de la pseudorage à l'état brisé, lesdits produits fournissant des antigènes qui sont sélectivement immunoréactifs avec l'anticorps du virus de la pseudorage.

2. Une composition de latex selon la revendication 1, qui est dispersée dans une suspension aqueuse.

3. Une composition de latex selon la revendication 1 ou 2, selon laquelle le rapport pondéral entre les produits du virus de la pseudorage à l'état brisé et les particules de latex est de 1:30 à 1:200.

4. Une composition de latex selon l'une quelconque des revendications précédentes qui inclut en outre l'albumine de sérum bovin, l'ovalbumine ou une autre protéine immunlogiquement inerte adsorbée sur les particules de latex.

5. Une composition de latex selon l'une quelconque des revendications précédentes, selon laquelle le latex est du latex de polystyrène.

6. Une composition de latex selon l'une quelconque des revendications précédentes, selon laquelle la particule de latex a un diamètre dans l'intervalle de 0,05 à 2 μm.

7. Une composition de latex selon l'une quelconque des revendications précédentes, selon laquelle les produits du virus de la pseudorage à l'état brisé sont adsorbés passivement sur les particules de latex, ou sont liés par covalence aux particules de latex.

8. Une composition de latex selon l'une quelconque des revendications précédentes, selon laquelle les produits du virus de la pseudorage à l'état brisé sont produits à partir du virus de la pseudorage brisé et solubilisé par un agent surfactant.

9. Un procédé pour détecter la présence de l'anticorps du virus de la pseudorage dans un échantillon biologique comprenant:

le mélangeage de l'échantillon biologique avec une composition de latex comprenant des produits du virus de la pseudorage à l'état brisé liés à des particules de latex dispersées dans une suspension liquide, lesdits produits du virus de la pseudorage fournissant des antigènes qui sont sélectivement immunoréactifs vis-à-vis des anticorps du virus de la pseudorage, comme revendiqué dans la revendication 1;

l'incubation de mélange de la composition de latex et de l'échantillon biologique pendant un temps suffisant pour permettre une immunoréaction; et

la détermination de la production d'une agglutination, cette agglutination indiquant la présence de l'anticorps du virus de la pseudorage dans l'échantillon biologique.

10. Un procédé selon la revendication 9, selon lequel l'échantillon biologique est du sérum ou du plasma de porc ou d'un autre animal.

11. Un procédé selon l'une quelconque des revendications précédentes, selon lequel l'échantillon biologique est prétraité par du kaolin, du talc, du charbon de bois ou de l'apatite pour éliminer les agglutinines non spécifiques et/ou dilué avec un diluant immunologiquement inerte avant le mélangeage avec la composition de latex.

**Revendications pour l'Etat contractant: AT**

1. Un procédé de préparation d'une composition de latex comprenant des particules discrètes de latex auxquelles sont liés des produits du virus de la pseudorage à l'état brisé, lesdits produits fournissant des antigènes qui sont sélectivement immunoréactifs vis-à-vis de l'anticorps du virus de la pseudorage, ledit procédé comprenant la mise en contact des particules discrètes de latex avec les produits du virus de la pseudorage à l'état brisé.

2. Un procédé selon la revendication 1, selon lequel les particules de latex sont dispersées dans un liquide aqueux.

3. Un procédé selon la revendication 1 ou 2, selon lequel le rapport pondéral entre les produits du virus de la pseudorage à l'état brisé et les particules de latex est de 1:30 à 1:200.

4. Un procédé selon l'une quelconque des revendications précédentes, qui comprend en outre l'adsorption de l'albumine de sérum bovin, de l'ovalbumine ou d'une autre protéine immunologiquement inerte sur les particules de latex après leur contact avec les produits du virus de la pseudorage à l'état brisé.

5. Un procédé selon l'une quelconque des revendications précédentes, selon lequel le latex est du latex de polystyrène.

6. Un procédé selon l'une quelconque des revendications précédentes, selon lequel la particule de latex a un diamètre dans l'intervalle de 0,05 à 2 µm.

7. Un procédé selon l'une quelconque des revendications précédentes, selon lequel les produits du virus de la pseudorage à l'état brisé sont adsorbés passivement sur les particules de latex ou sont liés par covalence aux particules de latex, et/ou les produits du virus de la pseudorage à l'état brisé sont produits par rupture et solubilisation du virus de la pseudorage par un agent surfactant.

8. Un procédé pour détecter la présence de l'anticorps du virus de la pseudorage dans un échantillon biologique comprenant:

le mélangeage de l'échantillon biologique avec une composition de latex comprenant des produits du virus de la pseudorage à l'état brisé liés aux particules de latex dispersés dans une suspension liquide, lesdits produits du virus de la pseudorage fournissant des antigènes qui sont sélectivement immunoréactifs avec l'anticorps du virus de la pseudorage, comme revendiqué dans la revendication 1;

l'incubation du mélange de la composition de latex et de l'échantillon biologique pendant un temps suffisant pour permettre une immunoréaction; et

la détermination de la production d'une agglutination, ladite agglutination indiquant la présence de l'anticorps du virus de la pseudorage dans l'échantillon biologique.

9. Un procédé selon la revendication 8, selon lequel l'échantillon biologique est du sérum ou du plasma de porc ou d'un autre animal.

10. Un procédé selon la revendication 8 ou 9, selon lequel l'échantillon biologique est prétraité par du kaolin, du talc, du charbon de bois ou de l'apatite pour éliminer les agglutinines non spécifiques et/ou dilué par un diluant immunologiquement inerte avant le mélangeage avec la composition de latex.